# EUROPÄISCHE PATENTSCHRIFT

(11) **EP 3 017 290 B1**
(45) Veröffentlichungstag und Bekanntmachung des Hinweises auf die Patenterteilung: **30.11.2016**
(21) Anmeldenummer: 14789977.7
(22) Anmeldetag: 21.06.2014
(51) Int. Cl.: G01N 21/01, G01N 21/03, G01N 33/49, G01N 21/80

(54) **DIFFUSIONSKAMMER ZUR ERMITTLUNG UNTERSCHIEDLICHER PARAMETER EINER WÄSSRIGEN SUBSTANZ**
DIFFUSION CHAMBER FOR ASCERTAINING DIFFERENT PARAMETERS OF AN AQUEOUS SUBSTANCE
CHAMBRE À DIFFUSION SERVANT À DÉTERMINER DIFFÉRENTS PARAMÈTRES D'UNE SUBSTANCE AQUEUSE

(30) Priorität: 01.07.2013 DE 102013011343
(43) Veröffentlichungstag der Anmeldung: 11.05.2016
(73) Patentinhaber: Alfred-Wegener-Institut Helmholtz-Zentrum für Polar- und Meeresforschung, 27570 Bremerhaven (DE)
(72) Erfinder: OELLERMANN, Michael, 28201 Bremen (DE); MARK, Felix, Christopher, 27574 Bremerhaven (DE); DUNKER, Erich, 27619 Schiffdorf (DE)
(86) Internationale Anmeldenummer: PCT/DE2014/000322
(87) Internationale Veröffentlichungsnummer: WO 2015/000453

(56) Entgegenhaltungen:
- US-A1- 2006 109 469
- S. ZIELINSKI ET AL.: "Temperature Effects on Hemocyanin Oxygen Binding in an Antartic Cephalopod", BIOL. BULL., Bd. 200, Februar 2001 (2001-02), Seiten 67-76, XP002733297, in der Anmeldung erwähnt
- TAN W ET AL: "Miniaturized fiber-optic chemical sensors with fluorescent dye-doped polymers", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, CH, Bd. 28, Nr. 2, 1. August 1995 (1995-08-01) , Seiten 157-163, XP027261054, ISSN: 0925-4005 [gefunden am 1995-08-01]
- M. Oellermann ET AL: "Simultaneous high-resolution pH and spectrophotometric recordings of oxygen binding in blood microvolumes", Journal of Experimental Biology, vol. 217, no. 9, 16 January 2014 (2014-01-16), pages 1430-1436, XP055156242, ISSN: 0022-0949, DOI: 10.1242/jeb.092726

## Beschreibung

Die Erfindung bezieht sich auf eine Diffusionskammer zur Ermittlung unterschiedlicher Parameter einer wässrigen Substanz mit einem vertikalen Zentralzylinder mit zumindest einer Probenkammer zur Aufnahme einer Substanzprobe, einem nadelförmigen pH-Messsensor mit einer die Substanzprobe kontaktierenden Messspitze, einem Spektrophotometer mit einer die Substanzprobe durchstrahlenden Glasfaseroptik und einer Gaszuführung in die Probenkammer zur veränderlichen Begasung der Substanzprobe während der Parameterermittlung.

Der pH-Wert ist ein Maß für den sauren oder basischen Charakter einer wässrigen Substanz. Diese kann in Abhängigkeit von ihrem aktuellen pH-Wert stark variierende Eigenschaften zeigen. Beispielsweise wirkt sich bei Blut der pH-Wert auf die Sauerstoffbindungseigenschaften von Blutpigmenten, wie Hämoglobin oder Hämocyanin, aus. Je geringer der pH-Wert ist, desto weniger Sauerstoff kann das Blutpigment über seine prosthetische Gruppe (d.h. Eisen oder Kupfer) binden. Diese Eigenschaft führt zu einer erleichterten Abgabe von Sauerstoff im Gewebe, wenn dort der Blut-pH-Wert durch stoffwechselbedingte Kohlendioxidabgabe sinkt (Bohr-Effekt). Wird umgekehrt in der Lunge Kohlendioxid abgeatmet, so steigt dort der pH-Wert des Blutes und somit die Aufnahmefähigkeit des Blutpigmentes für Sauerstoff. pH-Wert-Änderungen fließen in die Sauerstoff-Bindungskurven ein, die die Beziehung zwischen dem herrschenden Sauerstoff-Partialdruck und dem prozentualen Anteil des an das Blutpigment gebundenen Sauerstoffs (Blutpigmentsättigung) darstellen. Das Sauerstoffgefälle als Abnahme der Sauerstoff-Partialdrücke zwischen der Außenluft und den Körperzellen bestimmt die Aufnahme des Sauerstoffs. Eine optimale Sauerstoffaufnahme und -abgabe ermöglicht einen effizienten Sauerstofftransport und damit eine hohe Leistungsfähigkeit des Organismus. Mit Sauerstoff angereichertes Blut verändert seinen Farbton durch Konformationsänderungen der jeweiligen prosthetischen Gruppe bei Bindung von Sauerstoff. Beispielsweise zeigt oxygeniertes Hämoglobin einen helleren und kräftigeren Farbton als de-oxygeniertes Hämoglobin, oxygeniertes Hämocyanin hingegen färbt sich blau. Dies hat eine Veränderung des Absorptionsverhaltens der Substanzprobe bei Bestrahlung mit ultraviolettem oder sichtbarem Licht zur Folge. Durch simultane Messungen des pH-Werts und des AbsorptionsverhaItens zur Ermittlung der Sauerstoffbindungseigenschaften von Blut bei in-vivo- oder quasi-in-vivo-Untersuchungen können somit wichtige Aussagen über den aktuellen Zustand der Substanz bzw. ihres Trägers gewonnen werden. Dabei ist es aber insbesondere auch wichtig, mit nur geringen Probenvolumen der zu untersuchenden Substanz auskommen zu können, da die Menge des Probenmaterials oft stark limitiert ist (beispielsweise bei Kleinstorganismen).

### Stand der Technik

Aus der US 5 564 419 A ist eine Vorrichtung zur photometrischen in-vitro-Bestimmung des Sauerstoffgehalts in einer Blutprobe bekannt. Mit einer spritzenähnlichen Vorrichtung kann das Blut direkt aus dem Organismus gesogen und über einen Kanal geleitet werden. Dieser wird von Öffnungen in einem Träger gekreuzt, durch die Licht zur Absorptionsmessung eingestrahlt wird. Die Öffnungen sind mit einem Dichtring abgedichtet. Eine pH-Wert-Messung erfolgt jedoch nicht. Aus der EP 2 110 430 B1 ist eine Messzelle zur Messung des biochemischen Sauerstoffbedarfs bekannt, bei der das Ende eines Lichtwellenleiters von einem Spektrophotometer über ein Dichtelement mit der zu untersuchenden Substanz in Verbindung gebracht wird. Aus der US 5 056 520 ist ein nadelförmiger optischer Messsensor mit einem Lichtwellenleiter aus Silizium bekannt, mit dem der pH-Wert einer Blutprobe festgestellt werden kann. Der Lichtwellenleiter wird von einer Hülse geführt und endet mit der Messspitze in.einem Tropfen der zu untersuchenden Substanz.

Aus der Veröffentlichung "Method for Continous Registration of O2-Binding Curves of hemoproteins by Means of a Diffusion Chamber" von H. Sick und K. Gersonde (in Analytical Biochemistry 32, 362-376 (1969) ist eine Diffusionskammer zur photometrischen Messung des Absorptionsverhaltens von Blut unter verschiedenen Begasungen bekannt. In einem Zentralzylinder wird die zu untersuchende Blutprobe auf einem Plexiglasschlitten gelagert und mit Licht einer Quecksilberdampflampe durchstrahlt, das in einem Photodetektor detektiert wird. Der Plexiglasschlitten ist mit einem Dichtring gegenüber dem Zentralzylinder abgedichtet. Die Gasmischungen werden über eine Gaszufuhr in den Zentralzylinder eingeleitet. Eine pH-Wert-Messung erfolgt nicht.

Aus der Veröffentlichung "Oxygen evolution in a hypersaline crust: in situ photosynthesis quantification by microelectrode profiling and use of planar optode spots in incubation chambers" von J. Woelfel et al. (in Aquat Microb Ecol Vol.56:263-273, 2009) ist es bekannt, eine Sauerstoff-Mikrooptode mit Sensor-Spots und einer Faseroptik zur optischen Messung der Primärproduktion von Sauerstoff in der Arktis einzusetzen. Dabei wird die Sauerstoffentwicklung von bodenlebenden Diatomeen im Freiland in einer benthischen Kammer online über optische Fasern erfasst. Das Grundprinzip der optischen Sauerstoffmessung beruht auf Anregung eines spezifischen Fluoreszenzfarbstoffes (Indikatormolekül) durch Licht einer definierten Wellenlänge sowie dessen Fluoreszenzlöschung in Abhängigkeit von der Sauerstoffkonzentration, vergleiche URL Stand 20.06.2013 http://www.angewandteoekologie.unirostock.de/en/forschung3/analytik0/mikroopt/.

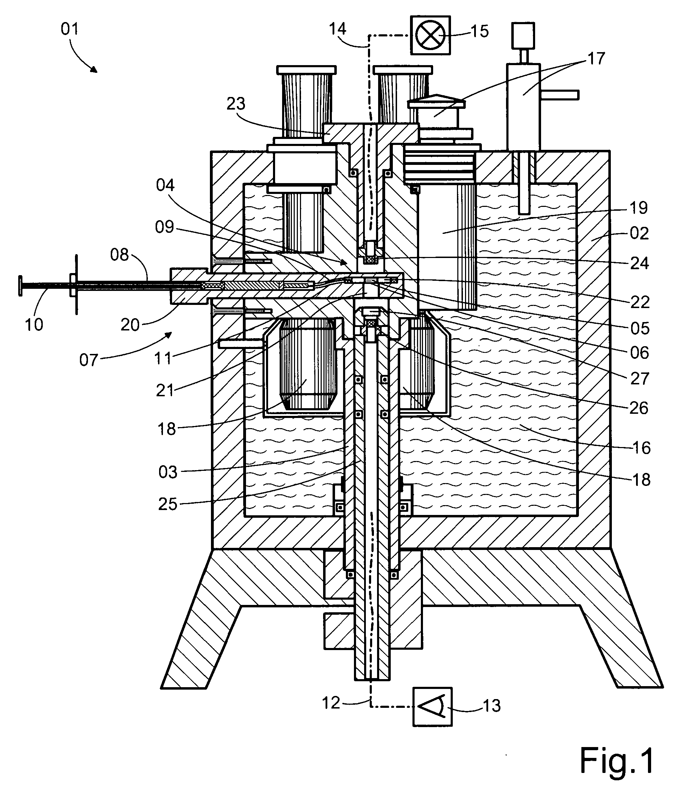

Der der Erfindung nächstliegende Stand der Technik wird in der Veröffentlichung "Temperature Effects on Hemocyanin Oxygen Binding in an Antartic Cephalopod" von S. Zielinski et al. (in Biol. Bull. 200:67-76 (February 2001) offenbart. Beschrieben wird eine Diffusionskammer mit einer modifizierten Quarzglasküvette zur simultanen Ermittlung von pH- und Absorptionsveränderungen von Blut eines Kopffüßers unter in-vitro-Bedingungen. Bei Zugabe in die Küvette verteilt sich das zu untersuchende Blut (400 µl) auf ein oberes und unteres Reservoir, das kontinuierlich mit Hilfe von Magnetrührern gemischt wird. Das Blut verläuft zudem in einem Bereich zwischen den beiden Reservoirs zu einer dünnen Schicht (0,45 mm), in der die Absorptionsmessung stattfindet. Die Messspitze einer Mikro-pH-Elektrode wird über einen oberen abdichtenden Deckel in die Blutprobe zur pH-Wert-Messung eingeführt. Eine zweite Öffnung im Deckel ermöglicht die Begasung mit einem während der Messung veränderbaren Gasgemisch (Sauerstoff, Kohlendioxid, Stickstoff). Die auftretende Absorptionsveränderung wird mit einem Spektrophotometer mit Glasfaseroptik gemessen. Der genaue konstruktive Aufbau wird in der Veröffentlichung nicht weiter dargestellt, vergleiche Figur 1 und Absatz "Analysis of oxygen binding".

Die Messprozedur wird jedoch ausführlich beschrieben. Die Analyse der Sauerstoffsättigung und Bindungseigenschaften von Blutpigmenten durch Messung der Lichtabsorption wird zum überwiegenden Teil mit Blutproben vorgenommen, die gepuffert wurden, um den pH-Wert zu stabilisieren. Puffer sind wässrige Lösungen einer schwachen Säure und ihrer konjugierten Base. Bei Zugabe von sauren (mit H₃O⁺-Ionen) und alkalischen Lösungen (OH⁻) werden die Hydroniumionen von der Pufferbase und die Hydroxidionen von der Puffersäure abgefangen (abgepuffert), sodass sich der pH-Wert bei dieser Zugabe nur sehr wenig ändert. Ein Puffer ist jedoch nur wirksam, wenn größere Mengen von Puffersäure und Pufferbase vorhanden sind. Die Pufferung beeinflusst jedoch nachweislich die Bindungseigenschaften des Blutpigments und gibt somit die tatsächlichen physiologischen Eigenschaften nicht immer wahrheitsgemäß wieder. Dieses Problem wird bei Pörtner (1990 ("An analysis of the effects of pH on oxygen binding by squid (Illex illecebrosus, Loligo pealei) haemocyanin", J. Exp. Biol. 150, 407), 1994 ("Coordination of metabolism, acid-base regulation and haemocyanin function in cephalopods", Mar. Fresh. Behav. Physiol. 25, 131-148)) und Zielinski et al. (2001, "Temperature effects on hemocyanin oxygen binding in an Antarctic cephalopod", Biol. Bull. (Woods Hole) 200, 67-76) dadurch gelöst, dass natives, ungepuffertes Blut bei konstantem Sauerstoffpartialdruck, aber variiertem pH-Wert untersucht wurde. Durch die Messung weiterer Bindungskurven bei verschiedenen Sauerstoffpartialdrücken konnten dann dieselben Parameter bestimmt werden wie bei der konventionellen Methodik mit den o.g. Nachteilen. Nachteilig bei der Methode von Pörtner und Zielinski et al. ist jedoch wiederum, dass ein hoher Probenverbrauch auftritt und die pH-Wert-Messungen an gepoolten Blutproben durchgeführt werden, sodass die Messwerte nicht eindeutig einzelnen Organismen zugeordnet werden können als auch statistisch nicht ausgewertet werden können.

### Aufgabenstellung

Ausgehend von dem zuletzt genannten Stand der Technik ist die Aufgabe für die vorliegende Erfindung darin zu sehen, die gattungsgemäße Diffusionskammer so weiterzuentwickeln, dass sowohl pH-Wert- als auch Absorptionsmessungen gleichzeitig auch an sehr kleinen Substanzproben durchgeführt werden können. Dabei sollen die Messungen möglichst schnell verlaufen bei gleichzeitig hoher Auflösung der Messergebnisse. Die erfindungsgemäße Lösung für diese Aufgabe ist dem Anspruch 1 zu entnehmen. Vorteilhafte Weiterbildungen der Erfindung werden in den Unteransprüchen aufgezeigt und im Folgenden im Zusammenhang mit der Erfindung näher erläutert.

Die erfindungsgemäße Diffusionskammer ist dadurch gekennzeichnet, dass radial in den Zentralzylinder gasdicht ein Probenschlitten eingeschoben ist, der eine zylindrische Öffnung und eine schräg verlaufende Nut aufweist. Oberhalb der zylindrischen Öffnung ist ein zylindrischer, die Probenkammer umschließender Dichtring auf dem Probenschlitten angeordnet. In die schräg verlaufende Nut im Probenschlitten ist erfindungsgemäß eine Kanüle eines spritzenartigen optischen Mikrosensors eingelegt. Dieser bildet den pH-Messsensor. Dabei ist in der Kanüle eine die Messspitze tragende Glasfaser verschiebbar gelagert. Außerdem ist die Kanüle durch den Dichtring gasdicht hindurchgeführt. Weiterhin ist die erfindungsgemäße Diffusionskammer dadurch gekennzeichnet, dass axial in den Zentralzylinder oberhalb der Probenkammer ein oberer zylindrischer Optikhalter mit einer lichtzuleitenden Glasfaser und einer oberen Kollimatorlinse und unterhalb der Probenkammer ein unterer zylindrischer Optikhalter mit einer lichtableitenden Glasfaser, einer unteren Kollimatorlinse und einem Abstandsring gasdicht eingeschoben sind. Dabei wird erfindungsgemäß durch den eingeschobenen Abstandsring sowohl der minimale Lichtweg von der Kollimatorlinse zur Probe eingehalten als auch der Dichtring auf dem Probenschlitten gegen den Zentralzylinder gedrückt und die Probenkammer dicht gegenüber Gasen aus der Umgebung der Diffusionskammer verschlossen.

Bei der erfindungsgemäßen Diffusionskammer wird erstmals bei einer Diffusionskammer ein optischer Mikrosensor - eine sogenannte "pH-Optode" - zur Messung des pH-Werts eingesetzt, durch die die Messwerte mit einer guten Messfrequenz und hohen Messgenauigkeit auf optischem Wege ermittelt werden. Alle bekannten Diffusionskammern messen den pH-Wert bislang extern in einer parallel begasten Blutprobe, auf elektrischem Wege mit pH-Elektroden, die einen größeren Platzbedarf haben und durch die größeren Sensordurchmesser auch ein größeres Probenvolumen benötigen. Ein weiterer Vorteil der pH-Optode besteht darin, dass über einen breiten Temperaturbereich (auch 0°C und kälter) stabil, genau und schnell gemessen werden kann. Herkömmliche pH-Elektroden messen im Gegensatz dazu bei kalter Umgebung nur äußerst träge und instabil. Die bei der Erfindung eingesetzte pH-Optode ist spritzenartig aufgebaut und verfügt über eine Kanüle, in der eine Glasfaser verschiebbar gelagert ist. Das freie Ende der Glasfaser trägt die Messspitze und kann einen Durchmesser 150 µm oder weniger haben. Entsprechend klein kann das Messvolumen der Substanzprobe sein, es kann 20 µl oder weniger betragen. Beispielsweise genügen 10 - 15 µl für die Substanzprobe - im Vergleich dazu werden bislang im Stand der Technik 400 µl bis 3 ml benötigt.

Eine besondere Herausforderung bei der erfindungsgemäßen Diffusionskammer ist die Integration einer solchen pH-Optode in den Messaufbau. Dazu ist bei der Erfindung ein spezieller Probenschlitten vorgesehen, der eine schräge Nut aufweist. Die Nut verläuft vom Körper der Kanüle schräg ansteigend zum Ende der Kanüle. Dadurch ist gewährleistet, dass die aus der Kanüle herausgeschobene Glasfaser mit der Messspitze in die Substanzprobe eintaucht und nicht darüber hinweg verläuft. Die Substanzprobe wird während der Messungen gezielt mit Gasmischungen bekannter Zusammensetzung (Partialdruck) begast. Dabei muss ausgeschlossen werden, dass versehentlich Umgebungsluft in den Gasstrom oder direkt in Kontakt mit der Substanzprobe gerät. Dafür ist bei der Diffusionskammer erfindungsgemäß vorgesehen, dass auf dem Probenschlitten ein Dichtring angeordnet ist, durch den die Kanüle gasdicht hindurchgeführt ist. Der Dichtring umschließt die Probenkammer, die oberhalb einer Öffnung im Probenschlitten gebildet ist. Die zu untersuchende Substanzprobe wird auf ein Glasplättchen aufgetragen, das über der Öffnung unterhalb des Dichtrings positioniert ist. Dadurch kann der Lichtstrahl des Spektrophotometers die Substanzprobe durchstrahlen und deren Absorption messen.

Eine weitere Herausforderung bei der erfindungsgemäßen Diffusionskammer stellt die Integration der Faseroptik des Spektrophotometers in den Messaufbau dar. Dieses wurde erfindungsgemäß durch zwei zylindrische Optikhalter gelöst, die ober- und unterhalb der Probenkammer angeordnet sind. Die beiden Optikhalter fixieren die Glasfasern von der Lichtquelle (oben) und zum Spektrophotometer (unten) und tragen die Sammellinsen sowie mehrere Dichtringe. Sie lassen sich einfach in den Zentralzylinder einschieben oder einschrauben und damit abdichten. Auf dem unteren Optikhalter ist noch zusätzlich ein Abstandsring angeordnet. Dieser drückt im eingeschobenen Zustand von unten gegen den eingeschobenen Probenschlitten und gewährleistet einen Mindestabstand von der Sammellinse zur Substanzprobe. Der Probenschlitten wiederum drückt den auf ihm angeordneten Dichtring gegen das Innere des Zentralzylinders und bewirkt so eine Abdichtung des Dichtrings im Zentralzylinder. Dadurch wird das Einströmen von die Messungen verfälschender Umgebungsluft in die Probenkammer während des Messvorgangs verhindert.

Durch die Verwendbarkeit einer kompakten hochauflösenden pH-Optode und eines kompakten hochauflösenden Spektrophotometers aufgrund der speziellen Integration der jeweiligen Faseroptik kann mit der Diffusionskammer nach der Erfindung eine sehr kompakte, transportable und leistungsfähige Messapparatur zur Verfügung gestellt werden, mit der zuverlässig und genau auch kleinste Substanzprobenvolumina, beispielsweise geringe Mengen Blut von sehr kleinen Organismen, hochaufgelöst gemessen werden können. Dabei können die Messwerte synchron an identischen Substanzproben ermittelt werden, sodass durch diese Konkordanz die Aussagekräftigkeit der Messergebnisse noch erhöht und die Anwendungspalette erweitert werden kann.

Weiter oben wurde bereits ausgeführt, dass es essenziell ist, dass die Messspitze in die Substanzprobe gelangt. Dazu wird die Kanüle der pH-Optode schräg in den Probenschlitten eingelegt. Dieser weist dazu eine schräge Nut auf. Um das Eintauchen der Messspitze am Ende der Glasfaser in die Substanzprobe noch zu verbessern, ist es vorteilhaft und bevorzugt, wenn die Kanüle zweifach winklig gebogen ist, sodass das zum Herausschieben der Glasfaser offene Ende der Kanüle parallel verschoben ist. Die Kanüle wird dadurch auf ein Niveau in der Mitte der zu untersuchenden Substanz angehoben. Die Glasfaser braucht dadurch nur noch ein kleines Stück aus der Kanüle herausgeschoben werden und kann sich nicht mehr absenken. Die Substanzprobe wird zur Absorptionsmessung von Licht durchstrahlt. Dabei ist sicherzustellen, dass keine Fremdkörper in den Lichtstrahl gelangen, die Licht absorbieren und zu Messfehlern führen würden. Solche Fehler bei der Absorptionsmessung können bei der Diffusionskammer nach der Erfindung bevorzugt und vorteilhaft dadurch verhindert werden, dass das zum Herausschieben der Glasfaser offene Ende der Kanüle bündig mit der Innenkontur des Dichtrings abschließt und das die aus der Kanüle herausgeschobene Glasfaser mit ihrer Messspitze in einen Randbereich der zu untersuchenden Substanzprobe eingetaucht ist. Weder die Kanüle noch die herausgeschobene Glasfaser beeinträchtigen so den einfallenden Lichtstrahl. Damit dies während der Messungen auch sicher gewährleistet bleibt, ist es bevorzugt und vorteilhaft, wenn der optische Mikrosensor durch eine schräge Verschraubung in einer geraden Nut im Probenschlitten fixiert ist. Nach dem Einlegen kann die leicht zugängliche Verschraubung betätigt und der optische Mikrosensor bzw. sein Spritzenkörper fixiert werden. Weiterhin ist es zur Sicherung von zuverlässigen Messergebnissen bevorzugt und vorteilhaft, wenn die Messspitze einen Durchmesser von unter 150 µm aufweist. Dadurch können zum einen sehr kleine Substanzprobenvolumina eingesetzt werden. Das zuverlässige Eintauchen der besonders kleinen Messspitze ist trotzdem sicher gewährleistet. Zum anderen absorbieren weder die kleine Messspitze noch die diese tragende Glasfaser Licht in einem solchen Anteil, dass die Messergebnisse verfälscht werden könnten.

Durch den möglichen Einsatz von sehr kleinen Substanzprobenvolumina bei der Diffusionskammer nach der Erfindung kann bevorzugt und vorteilhaft in der Probenkammer ein Messvolumen von unter 20 µl für die zu untersuchende Substanzprobe eingesetzt werden. Damit können beispielsweise mehr Messungen pro Blutprobe durchgeführt oder auch Blutproben von kleinsten Organismen mit limitierten Blutmengen untersucht werden. Weiterhin können dabei auch Messprofile, beispielsweise die Ermittlung von pH-Wert-Gradienten, über die Veränderung der anderen Messparametern, beispielsweise Variationen in der Gaszusammensetzung, Temperatur, Anregungs- und Detektionswellenlänge, erstellt werden, wenn bevorzugt und vorteilhaft der optische Mikrosensor eine Messsteuerung zur Ermittlung von zumindest einem Messwert pro Sekunde aufweist. Ebenfalls besonders vorteilhaft und bevorzugt bei der Erstellung von genauen Messprofilen ist es, wenn das Spektrophotometer eine Messsteuerung zur Ermittlung von zumindest 1000 Spektren pro Sekunde in einem kontinuierlichen Wellenlängenbereich zwischen 200 nm und 1100 nm aufweist. Dabei kann jedes Spektrum in beispielsweise 2048 Datenpunkte aufgelöst sein. Diese hohe Datendichte bietet sehr viele Details über das gesamte Spektrum, was beispielsweise bei Nicht-Modellorganismen oder abnormen Blutproben von Vorteil ist. Derartige Spektrophotometer und auch pH-Optoden sind kommerziell erhältlich, vergleiche Ausführungsbeispiele. Durch solche leistungsfähigen Messgeräte, die flexibel und kompakt sind und daher auch Feldversuche ermöglichen, wird die Datendichte beispielsweise pro ermittelter Bindungskurve deutlich erhöht. Beispielsweise können bei einer zweistündigen Messdauer 7.200 Datenpunkte bei einer nur geringen Integrationszeit ermittelt werden. In bekannten Verfahren können im Vergleich dazu in der Regel nur 6 bis 20 Datenpunkte ermittelt werden. Eine derart hohe Aufzeichnungsrate kann einerseits dazu genutzt werden, durch Mittelung das instrumentelle Rauschen deutlich zu verringern. Andererseits können nahezu kontinuierliche Bindungskurven ermittelt werden, wodurch neue, teilweise parametrisch sehr eng begrenzte Bindungseigenschaften aufgedeckt werden können. Als Beispiel sei die Kurvenasymmetrie der Bindungskurven in Abhängigkeit vom Sauerstoffpartialdruck genannt.

Weiter oben wurde bereits ausgeführt, dass bei der Diffusionskammer nach der Erfindung die Flexibilität bezüglich der Messparameter von Vorteil ist. Die Erfindung ermöglicht durch ihren besonderen konstruktiven Aufbau den Einsatz spezieller hochauflösender, fasergebundender pH-Optoden und Spektrophotometer. Insbesondere ist es auch vorteilhaft und bevorzugt, wenn das Spektrophotometer eine Halogen- oder Lumineszenz-Lichtquelle zur Bestrahlung der zu untersuchenden Substanzprobe aufweist. Dadurch können mit der Diffusionskammer nach der Erfindung auch kolorimetrische Assays oder Fluoreszenz- oder Lumineszenzassay erstellt werden. Dies gilt beispielsweise für Zellsuspensionen, bei denen beispielsweise die NADH-, Kalzium- und ATP-, Sauerstoff-Radikal- oder Caspase-Aktivität bei gleichzeitiger Messung des pH-Werts und zellulär bzw. physiologisch relevanter Gaszusammensetzung gemessen werden soll. Für Fluoreszenzmessungen muss dann lediglich eine LED-Lichtquelle, die bei einer einzelnen definierten Wellenlänge Licht emittiert, über einen Y-Wellenleiter an das optische Fasersystem des Spektrophotometers angeschlossen werden. Ein Austausch des Spektrophotometers ist nicht erforderlich. Weiterhin kann vorteilhaft und bevorzugt der Probenkammer über die Gaszufuhr eine einstellbare Gasmischung, beispielsweise aus Stickstoff, Sauerstoff und Kohlendioxid, zugeführt sein, um die oben genannte Parametervariation der Umgebung der zu untersuchenden Substanzprobe zu ermöglichen.

Die Flexibilität der Diffusionskammer nach der Erfindung bezüglich ihrer Anwendbarkeit auf unterschiedliche Messprobleme kann noch weiter verbessert werden, wenn bevorzugt und vorteilhaft in die Probenkammer weitere Messspitzen von weiteren optischen Mikrosensoren, beispielsweise zur Messung des O₂-, CO₂- oder NO-Gehalts, oder von chemischen Mikrosensoren, beispielsweise zur Detektion von farbsensitiven Verbindungen, der zu untersuchenden Substanzprobe eingeführt sind. Durch die Verfügbarkeit von O₂-, CO₂- oder NO-Optoden, die am Markt erhältlich sind und ohne Weiteres in den konstruktiven Aufbau der Diffusionskammer nach der Erfindung integriert werden können, können neben dem pH-Wert noch weitere Parameter ermittelt werden (O₂-, CO₂- oder NO-Gehalt). Dadurch kann die Bandbreite der Einsatzmöglichkeiten der Diffusionskammer nach der Erfindung noch weiter vergrößert werden. Zudem ist es möglich, beispielsweise chemische Messungen, bei denen beispielsweise farbsensitive Verbindungen gemessen werden, mit der gleichzeitigen pH-Wert-Messung mit der Diffusionskammer nach der Erfindung zu verbinden. Für die zu untersuchende Substanzprobe, bevorzugt und vorteilhaft eine Substanz biologischen Ursprungs, beispielsweise Blut oder eine Zellsuspension, ist es dabei von Vorteil und bevorzugt, wenn die Probenkammer bzw. das Gehäuse, das die Probenkammer umgibt, mit destilliertem oder mit Glykol angereichertem Wasser temperiert wird. Weitere vorteilhafte Modifikationen und Erläuterungen zu den genannten Ausführungsformen der Diffusionskammer nach der Erfindung sind den nachfolgend beschriebenen Ausführungsbeispielen zu entnehmen.

### Ausführungsbeispiele

Die Diffusionskammer nach der Erfindung wird im Folgenden anhand der schematischen, nicht maßstäblichen Figuren zum besseren Verständnis noch weitergehend erläutert. Im Einzelnen zeigt die
- Figur 1: eine Ausführungsform der Diffusionskammer im schematischen Querschnitt,
- Figur 2: ein Detail der Ausführungsform der Diffusionskammer,
- Figur 3: eine Ausführungsform des Probenschlittens im Detail,
- Figur 4: eine Ausführungsform des oberen Linsenhalters im Detail,
- Figur 5: eine Ausführungsform des unteren Linsenhalters im Detail,
- Figur 6A: verschiedene Sauerstoffbindungskurven, die mit der Diffusionskammer nach der Erfindung erstellt wurden und
- Figur 6B: den zeitlichen Verlauf der Veränderung des Blut-pH-Werts und der Absorption bei 294 nm und 348 nm einer Hämocyaninhaltigen Blutprobe.

In der Figur 1 ist die Diffusionskammer 01 nach der Erfindung schematisch im Querschnitt dargestellt. In einem Gehäuse 02 befindet ein vertikaler Zentralzylinder 03, in dessen Mittenbereich eine Probenkammer 04 zur Aufnahme einer wässrigen Substanzprobe 05 angeordnet ist. Die Substanzprobe 05 ist auf einer Glasplatte 06 (rechteckiges Hämatokritgläschen, dickeres Deckgläschen) in dünner Verteilung aufgetragen. An der linken Seite des Gehäuses 02 ist ein optischer Mikrosensor 07 (pH-Optode) mit einem Spritzenkörper 08, einem Stößel 10 und einer Kanüle 11 angeordnet, in der eine Glasfaser 09 geführt ist. Im unteren Bereich des Gehäuses 02 verläuft mittig im Zentralzylinder 03 eine Glasfaser 12, die zu einem Spektrophotometer 13 führt. Im oberen Bereich des Gehäuses 02 verläuft mittig im Zentralzylinder 03 eine weitere Glasfaser 14, die zu einer Lichtquelle 15 führt.

Das Gehäuse 02 ist mit einem Wasserreservoir 16 gefüllt, dessen Temperatur über ein Thermostat (nicht gezeigt) konstant gehalten wird. Ein Gaseinlassverteiler 17 (zum Anschluss mehrerer Leitungen für unterschiedliche Gasgemische) ist oben auf dem Gehäuse 02 angeordnet. Von dort wird das jeweilig aktuell zugeführte Gasgemisch, das über eine Gasmischpumpe (nicht gezeigt) auf die gewünschten Gasverhältnisse vorgemischt wird, dauerhaft und in einem gleichmäßigen Gasstrom während des gesamten Messzyklus eingeleitet wird, über das temperierte Wasserreservoir 16 zu Gasbefeuchtern 18 geleitet. Hierbei handelt es sich um Glaszylinder, in denen das zugeführte trockene Gasgemisch mit Wasser gesättigt und auf die Messtemperatur equilibriert wird. Dadurch wird die Substanzprobe beim Begasen nicht ausgetrocknet und ändert auch nicht ihre Messtemperatur. In den Gasbefeuchtern 18 wird das Gasgemisch durch eine poröse Membran, über der eine kleine Wassersäule ruht, getrieben. Dadurch entstehen feine Gasbläschen, die sich schnell mit Wasserdampf sättigen. Von den Gasbefeuchtern 18 strömt das feuchte Gasgemisch in einen Gasverteiler 19, der das Gasgemisch dann kontinuierlich in die Probenkammer 04 und über die Substanzprobe 05 leitet.

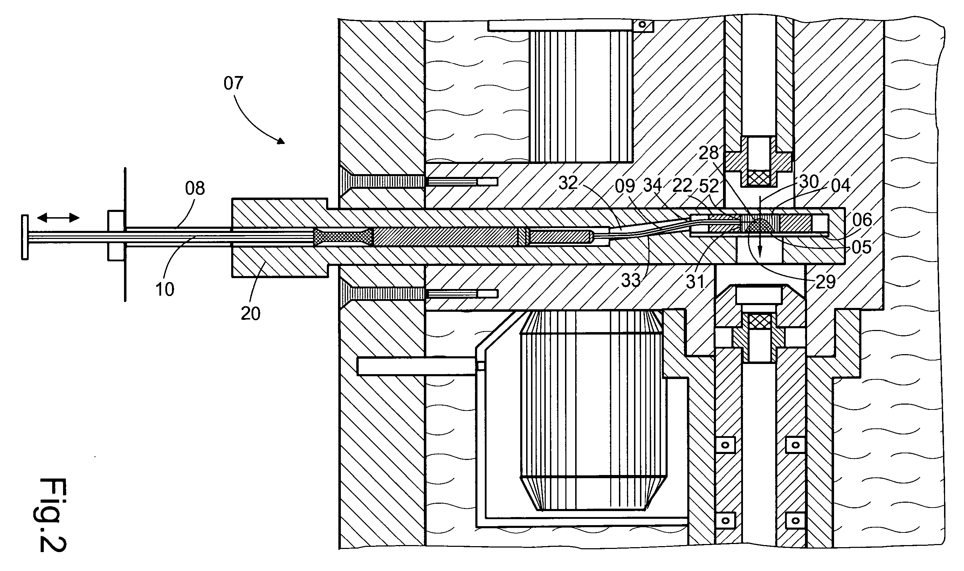

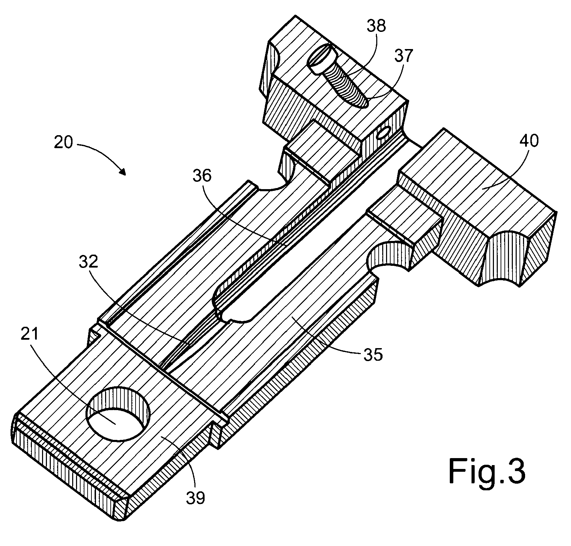

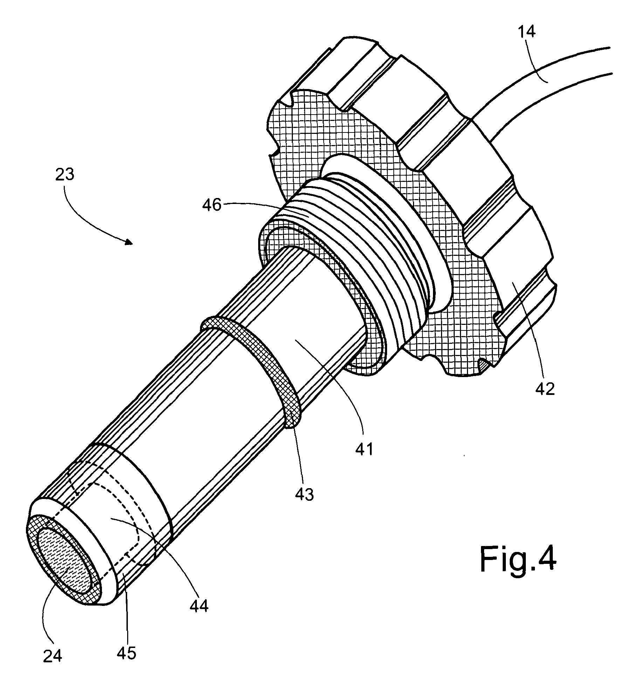

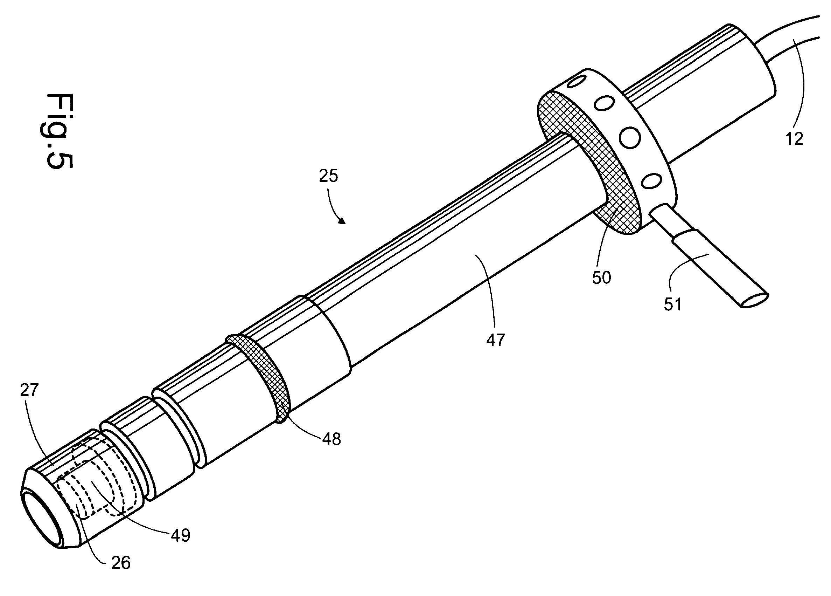

Radial in den Zentralzylinder 03 ist gasdicht ein Probenschlitten 20 eingeschoben, der eine zylindrische Öffnung 21 aufweist. Oberhalb dieser ist ein zylindrischer Dichtring 22 angeordnet, der die Probenkammer 04 gasdicht umschließt. Weitere Details sind den Figuren 2, 3 zu entnehmen. Axial ist in den Zentralzylinder 03 oberhalb der Probenkammer 04 ein oberer zylindrischer Optikhalter 23 mit der lichtzuleitenden Glasfaser 14 und einer oberen Kollimatorlinse 24 und unterhalb der Probenkammer 04 ein unterer zylindrischer Optikhalter 25 mit der lichtableitenden Glasfaser 12, einer unteren Kollimatorlinse 26 und einem Abstandsring 27 gasdicht eingeschoben. Zu erkennen ist, dass der eingeschobene Abstandsring 27 den Probenschlitten 20 und den darauf angeordneten Dichtring 22 gegen die Innenkontur des Zentralzylinders 03 drückt und dadurch die Probenkammer 04 während der Messungen gegen Eindringen von messverfälschender Umgebungsluft schützt. Weitere Details sind den Figuren 4, 5 zu entnehmen.

Ein möglicher Messzyklus mit der Diffusionskammer 01 sieht folgendermaßen aus:
- 15µl Substanzprobe 05 (beispielsweise Blut) werden auf die Glasplatte 06 des Probenschlittens 20 aufgetragen und dann in die Probenkammer 04 geschoben
- anschließend wird die Substanzprobe 05 mit einem 100% O₂-Gasstrom begast, bis sich die Absorption der Substanzprobe 05 nicht mehr verändert (= 100% Kalibrierung)
- anschließend wird der O₂-Gasstrom konstant auf z.B. 21 kPa (Partialdruck PO₂ während des Messzyklus) eingestellt und gewartet, bis die Absorption der Substanzprobe 05 wieder stabil ist und sich nicht mehr verändert.
- anschließend wird ein CO₂-Gasstrom schrittweise (oder kontinuierlich) erhöht, um den pH-Wert der Substanzprobe 05 soweit zu senken, bis sich die Absorption der Substanzprobe 05 nicht weiter erniedrigt (CO₂ löst sich in der Substanzprobe 05 und erzeugt über die Bildung von Kohlensäure die pH-Wert-Erniedrigung),
- abschließend wird mit einem 100% N₂-Gasstrom begast, um den 0% Kalibrierungswert (der Absorption der Substanzprobe 05) zu bestimmen
- während des gesamten Messzyklus werden der pH-Wert mit der pH-Optode 07) und die Absorption der Substanzprobe 05 (mit dem Spektrophotometer 13) mit einer größtmöglichen Messfrequenz gemessen, um aus den gewonnenen Datensätzen möglichst hochaufgelöste und damit besonders aussagekräftige Sauerstoff-Bindungskurven ermitteln zu können.

In der Figur 2 ist im Detail der Probenschlitten 20 mit der pH-Optode 07 dargestellt. Zu erkennen sind der Spritzenkörper 08, der Stößel 10 und die Kanüle 11. In der Kanüle 11 ist die optische Glasfaser 09 geführt. Sie trägt eine Messspitze 28, die beispielsweise mit einem Indikatormolekül beschichtet ist (vergleiche Beschreibungseinleitung). Über eine Bewegung (Doppelpfeil) des Stößels 10 wird die Glasfaser 09 und damit die Messspitze 28 radial verschoben und aus dem offenen Ende 52 der Kanüle 11 herausgeschoben. Dadurch gelangt die Messspitze 28 in einen Randbereich 29 der Substanzprobe 05 und stört den Strahlengang 30 zur Absorptionsmessung nicht. Die Kanüle 11 ist gasdicht durch den Dichtring 22 hindurchgeschoben und endet an dessen Innenkontur 31 bündig mit dem Dichtring 22. Somit wird auch durch die Kanüle 11 der Strahlengang 29 nicht gestört. In der Figur 2 ist noch zu erkennen, dass der Probenschlitten 20 eine Nut 32 aufweist, die schräg ansteigend zur Probenkammer 04 verläuft. Dadurch und durch eine zweifach winklige Biegung 33, 34 der Kanüle 11 wird erreicht, dass die Messspitze 28 auch zuverlässig in den Randbereich 29 der Substanzprobe 05 eintaucht und nicht darüber hinwegragt.

Als pH-Optode 07 mit einer sehr kleinen Messspitze 28 (150 µm) ist beispielsweise der kommerziell erhältliche glasfasergebundene pH-Microsensor der Firma PreSens einsetzbar, vergleiche PreSens GmbH, www.presens.de/ products/brochures/category/sensor-probes/brochure/ph-microsensors.html, Stand 23.06.2013. diese pH-Optode ist temperaturstabil und mit einer Messung pro Sekunde auch sehr schnell. Als Spektrophotometer 13 ist beispielsweise das kommerziell erhältliche glasfasergebundene Spektrophotometer der Firma OceanOptics einsetzbar, vergleiche OceanOptics, http://www.oceanoptics.com/ products/usb2000+.asp, Stand 23.06.2013. Dieses Spektrophotometer in Kombination mit einer Deuterium- und Halogen-Lichtquelle 15 (DT-Mini-2-GS, OceanOptics) zeichnet ein breites kontinuierliches Spektrum auf (200-1100 nm) mit einer Auflösung von 2048 Datenpunkten pro Spektrum auf. Diese hohe Datendichte bietet sehr viele Details über das gesamte Spektrum. Weiterhin kann das Spektrophotometer bis zu 1000 Spektren pro Sekunde aufzeichnen. Zusammen mit der ebenfalls hohen Datenmenge durch die pH-Optode wird die Datenmenge deutlich erhöht gegenüber bekannten Diffusionskammern.

Die Figur 3 zeigt den Probenschlitten 22 im Detail. Zu erkennen ist ein Schlittenkörper 35, in den die schräge Nut 32 eingefräst ist. Eine gerade Nut 36 ist zum Einlegen des Spritzenkörpers 08 vorgesehen. Weiterhin ist eine schräge Bohrung 37 für eine schräge Verschraubung 38 des Spritzenkörpers 08 im eingelegten Zustand vorgesehen. Im vorderen Bereich trägt der Schlittenkörper 35 eine rechteckige Auflagefläche 39 zum Auflegen der Glasplatte 06 mit der Substanzprobe 05. In der rechteckigen Auflagefläche 39 ist die zylindrische Öffnung 21 angeordnet, durch die während des Messens der Strahlengang 30 fällt. Weiterhin weist der Schlittenkörper 35 einen Knebelgriff 40 zum einfachen Ein- und Ausschieben des Probenschlittens 22 in den Zentralzylinder 03 auf.

Die Figur 4 zeigt im Detail den oberen Optikhalter 23 mit der lichtzuleitenden Glasfaser 14. Zu erkennen ist ein oberer Halterkörper 41 mit einem Handrad 42 und einem in eine Nut eingelegten Dichtungsring 43. Am unteren Ende ist eine Fassung 44 mit der oberen Kollimatorlinse 24 eingeschraubt und mit einer Abdeckkappe 45 gesichert. Der obere Optikhalter 23 wird mittels eines Gewindes 46 in den Zentralzylinder 03 eingeschraubt.

Die Figur 5 zeigt im Detail den unteren Optikhalter 25 mit der lichtableitenden Glasfaser 12. Zu erkennen ist ein unterer Halterkörper 47 mit einem in einer Nut eingelegten Dichtungsring 48. Am oberen Ende ist eine Fassung 49 mit der unteren Kollimatorlinse 26 eingeschraubt und mit dem Abstandsring 27 über ein Gewinde fixiert. Der untere Optikhalter 25 wird einfach in den Zentralzylinder 03 eingeschoben und hält durch die Dichtungsringe 48. Weiterhin weist er noch ein Justiergewinde 50 auf, das mit einem Knebel 51 betätigbar ist und der Fokussierung der unteren Kollimatorlinse 26 dient. Im eingeschobenen Zustand wird über den Abstandsring 27 der Probenschlitten 20 bzw. der Dichtring 22 gegen den Zentralzylinder 03 nach oben gedrückt und dadurch die Probenkammer 04 abgedichtet. Außerdem wird durch den Abstandsring 27 ein Minimalabstand zwischen der unteren Kollimatorlinse 26 und der Substanzprobe 05 eingehalten, der den Strahlengang 30 optimal auf die Substanzprobe 05 fokussiert.

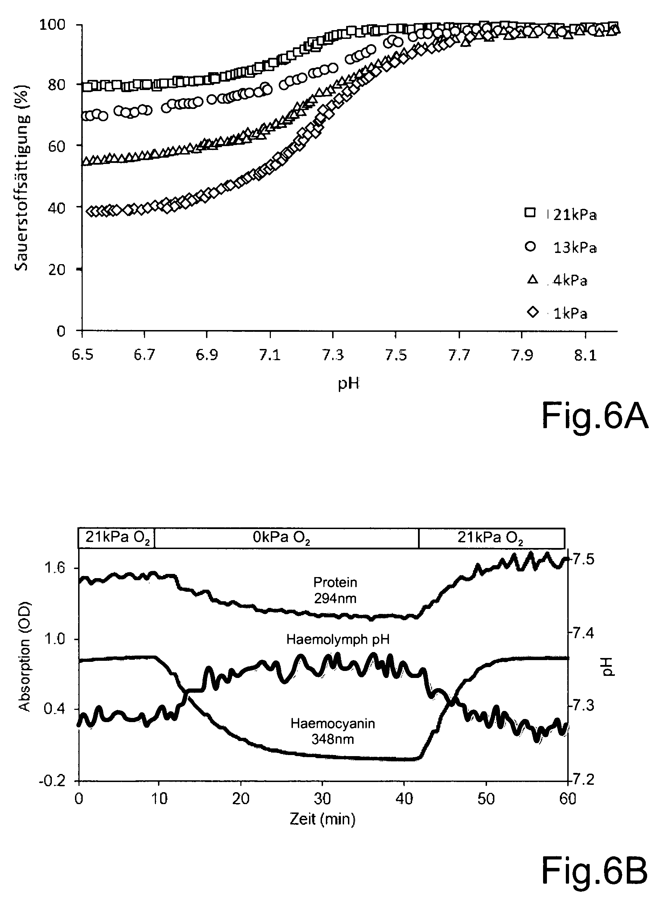

In der Figur 6A sind abschließend zur Unterstützung der guten erreichbaren Auflösung der Diffusionskammer 01 nach der Erfindung verschiedene ermittelte Sauerstoffbindungskurven vom Hämocyanin-haltigem Blut des antarktischen Kraken *Pareledone charcoti* dargestellt, gemessen bei einer Temperatur von 0°C. Dabei zeigt sich, dass zunächst mit nur insgesamt 60 µl Probenvolumen (4 x 15 µl) Messungen bei vier verschiedenen Sauerstoffpartialdrücken durchgeführt werden konnten vom Blut eines einzigen Kraken, von dem allgemein nur maximal 200 µl Blut gewonnen werden können. Die hohe Auflösung der Bindungskurven offenbarte dabei eine Asymmetrie der Bindungskurven in Abhängigkeit vom Sauerstoffpartialdruck. Dieses Phänomen wurde in vorangegangenen Studien aufgrund von nur wenigen Datenpunkten und Kurveninterpolation unter Annahme eines symmetrischen Kurvenverlaufes bislang nicht detektiert und weist auf komplexere Sauerstoffbindungseigenschaften hin als bisher angenommen.

Die Messungen belegen zudem deutlich in Figur 6B, dass die Oxygenierung bzw. De-Oxygenierung von Hämocyanin einhergeht mit einer Abgabe bzw. Aufnahme von Kohlendioxid, reflektiert durch den Abfall bzw. Anstieg des pH-Wertes der analysierten Blutprobe. Die breite spektrale Aufzeichnung offenbarte außerdem, dass die maximale Proteinabsorption von Kopffüßer-Hämocyanin nicht - wie bisher angenommen - bei 280 nm, sondern bei 294 nm liegt und zudem eine Abhängigkeit zwischen der Proteinabsorption und dem Oxygenierungsgrad des Blutpigmentes besteht. Diese neuen Erkenntnisse müssen bei zukünftigen Messungen zur Proteinbestimmung von Blutproben beachtet werden.

### Bezugszeichenliste

- 01: Diffusionskammer
- 02: Gehäuse
- 03: Zentralzylinder
- 04: Probenkammer
- 05: Substanzprobe
- 06: Glasplatte
- 07: optischer Mikrosensor (pH-Optode)
- 08: Spritzenkörper
- 09: Glasfaser
- 10: Stößel
- 11: Kanüle
- 12: lichtableitende Glasfaser
- 13: Spektrophotometer
- 14: lichtzuleitende Glasfaser
- 15: Lichtquelle
- 16: Wasserreservoir
- 17: Gaseinlassverteiler
- 18: Gasbefeuchter
- 19: Gasverteiler
- 20: Probenschlitten
- 21: zylindrische Öffnung
- 22: Dichtring
- 23: oberer zylindrischer Optikhalter
- 24: obere Kollimatorlinse
- 25: unterer zylindrischer Optikhalter
- 26: untere Kollimatorlinse
- 27: Abstandsring
- 28: Messspitze
- 29: Randbereich
- 30: Strahlengang
- 31: Innenkontur
- 32: schräge Nut
- 33: winklige Biegung
- 34: winklige Biegung
- 35: Schlittenkörper
- 36: gerade Nut
- 37: schräge Bohrung
- 38: Verschraubung
- 39: Auflagefläche
- 40: Knebelgriff
- 41: oberer Halterkörper
- 42: Handrad
- 43: Dichtungsring
- 44: Fassung
- 45: Abdeckkappe
- 46: Gewinde
- 47: unterer Halterkörper
- 48: Dichtungsring
- 49: Fassung
- 50: Justiergewinde
- 51: Knebel
- 52: offenes Ende von 11

## Patentansprüche

1. Diffusionskammer (01) zur Ermittlung unterschiedlicher Parameter einer wässrigen Substanz mit einem vertikalen Zentralzylinder (03) mit zumindest einer Probenkammer (04) zur Aufnahme einer Substanzprobe (05), einem nadelförmigen pH-Messsensor (07) mit einer die Substanzprobe kontaktierenden Messspitze (28), einem Spektrophotometer (13) mit einer die Substanzprobe durchstrahlenden Glasfaseroptik (12,14) und einer Gaszuführung (17) in die Probenkammer zur veränderlichen Begasung der Substanzprobe während der Parameterermittlung, **dadurch gekennzeichnet, dass**
• radial in den Zentralzylinder (03) gasdicht ein Probenschlitten (20) eingeschoben ist, der eine zylindrische Öffnung (21), oberhalb der ein zylindrischer, die Probenkammer (04) umschließender Dichtring (22) auf dem Probenschlitten (20) angeordnet ist, und eine schräg verlaufende Nut (32) aufweist, in die eine Kanüle (11) des als spritzenartiger optischer Mikrosensor (07) ausgebildeten pH-Messsensors eingelegt ist, wobei in der Kanüle (11) eine die Messspitze (28) tragende Glasfaser (09) verschiebbar gelagert ist und die Kanüle (11) durch den Dichtring (22) gasdicht hindurchgeführt ist, und dass
• axial in den Zentralzylinder (03) oberhalb der Probenkammer (04) ein oberer zylindrischer Optikhalter (23) mit einer lichtzuleitenden Glasfaser (14) und einer oberen Kollimatorlinse (24) und unterhalb der Probenkammer (04) ein unterer zylindrischer Optikhalter (25) mit einer lichtableitenden Glasfaser (12), einer unteren Kollimatorlinse (26) und einem Abstandsring (27) gasdicht eingeschoben sind, wobei durch den eingeschobenen Abstandsring (27) der Dichtring (22) auf dem Probenschlitten (20) gegen den Zentralzylinder (03) gedrückt und die Probenkammer (04) dicht gegenüber Gasen aus der Umgebung der Diffusionskammer (01) verschlossen sowie ein Minimalabstand zwischen der unteren Kollimatorlinse (26) und Substanzprobe (05) eingehalten ist.

2. Diffusionskammer nach Anspruch 1,
**dadurch gekennzeichnet, dass**
die Kanüle (11) zweifach winklig (33, 34) gebogen ist, sodass das zum Herausschieben der Glasfaser (09) offene Ende (52) der Kanüle (11) parallel verschoben ist.

3. Diffusionskammer nach Anspruch 1 oder 2,
**dadurch gekennzeichnet, dass**
das zum Herausschieben der Glasfaser (09) offene Ende (52) der Kanüle (11) bündig mit der Innenkontur (31) des Dichtrings (22) abschließt und die aus der Kanüle (11) herausgeschobene Glasfaser (09) mit ihrer Messspitze (28) in einen Randbereich (29) der zu untersuchenden Substanzprobe (05) eingetaucht ist.

4. Diffusionskammer nach einem oder mehreren der vorangehenden Ansprüche,
**dadurch gekennzeichnet, dass**
der optische Mikrosensor (07) durch eine schräge Verschraubung (38) in einer geraden Nut (36) im Probenschlitten (20) fixiert ist.

5. Diffusionskammer nach einem oder mehreren der vorangehenden Ansprüche,
**dadurch gekennzeichnet, dass**
die Messspitze (28) einen Durchmesser von 150 µm oder weniger aufweist.

6. Diffusionskammer nach Anspruch 5,
**dadurch gekennzeichnet, dass**
in der Probenkammer (04) ein Messvolumen von 20 µl oder weniger für die zu untersuchende Substanzprobe (05) eingesetzt ist.

7. Diffusionskammer nach einem oder mehreren der vorangehenden Ansprüche,
**dadurch gekennzeichnet, dass**
der optische Mikrosensor (07) eine Messsteuerung zur Ermittlung von zumindest einem Messwert pro Sekunde aufweist.

8. Diffusionskammer nach einem oder mehreren der vorangehenden Ansprüche,
**dadurch gekennzeichnet, dass**
das Spektrophotometer (13) eine Messsteuerung zur Ermittlung von zumindest 1000 Spektren pro Sekunde in einem kontinuierlichen Wellenlängenbereich zwischen 200 nm und 1100 nm aufweist.

9. Diffusionskammer nach einem oder mehreren der vorangehenden Ansprüche,
**dadurch gekennzeichnet, dass**
das Spektrophotometer (13) eine Halogen- oder Lumineszenz-Lichtquelle (15) zur Bestrahlung der zu untersuchenden Substanzprobe (05) aufweist.

10. Diffusionskammer nach einem oder mehreren der vorangehenden Ansprüche,
**dadurch gekennzeichnet, dass**
die Probenkammer (04) mit destilliertem oder mit Glykol angereichertem Wasser temperiert ist.

11. Diffusionskammer nach einem oder mehreren der vorangehenden Ansprüche,
**dadurch gekennzeichnet, dass**
der Probenkammer (04) über die Gaszufuhr (17) eine einstellbare Gasmischung, beispielsweise aus Stickstoff, Sauerstoff und Kohlendioxid, zugeführt ist.

12. Diffusionskammer nach einem oder mehreren der vorangehenden Ansprüche,
**dadurch gekennzeichnet, dass**
in die Probenkammer (04) weitere Messspitzen von weiteren optischen Mikrosensoren, beispielsweise zur Messung des O₂-, CO₂- oder NO-Gehalts, oder von chemischen Mikrosensoren, beispielsweise zur Detektion von farbsensitiven Verbindungen, der zu untersuchenden Substanzprobe (05) eingeführt sind.

13. Diffusionskammer nach einem oder mehreren der vorangehenden Ansprüche,
**dadurch gekennzeichnet, dass**
für die Substanzprobe (05) eine Substanz biologischen Ursprungs, beispielsweise Blut oder eine Zellsuspension, ausgewählt ist.

## Claims

1. A diffusion chamber (01) for ascertaining different parameters of an aqueous substance with a vertical central cylinder (03) with at least one sample chamber (04) for receiving a substance sample (05), a needle-shaped pH measuring sensor (07) with a measuring tip (28) making contact with the substance sample, a spectrophotometer (13) with glass fibre optics (12, 14) radiating light through the substance sample and a gas supply (17) into the sample chamber for the variable admission of gas to the substance sample during the ascertainment of the parameters,
**characterised in that**
• a sample slide (20) is inserted radially in a gas-tight manner into the central cylinder (03), which sample slide comprises a cylindrical opening (21), above which a cylindrical sealing ring (22) surrounding the sample chamber (4) is arranged on the sample slide (20), and a diagonal groove (32), into which a cannula (11) of the pH measuring sensor constituted as a syringe-like optical microsensor (07) is inserted, wherein a glass fibre carrying the measuring tip (28) is located displaceably in the cannula (11) and the cannula (11) is passed through the sealing ring (22) in a gas-tight manner, and that
• there are inserted axially in a gas-tight manner into the central cylinder (03), above the sample chamber (04), an upper cylindrical optical system holder (23) with a light-supplying glass fibre (14) and an upper collimator lens (24) and, beneath the sample chamber (04), a lower cylindrical optical system holder (25) with a light-removing glass fibre (12), a lower collimator lens (26) and a spacer ring (27), wherein the sealing ring (22) on the sample slide (20) is pressed against the central cylinder (03) by the inserted spacer ring (27) and the sample chamber (04) is sealed tight against gases from the surroundings of the diffusion chamber (01) and a minimum distance is maintained between the lower collimator lens (26) and the substance sample (05).

2. The diffusion chamber according to claim 1,
**characterised in that**
the cannula (11) is bent twice at an angle (33, 34), so that the open end (52) of the cannula (11) for pushing out the glass fibre (09) is displaced in a parallel manner.

3. The diffusion chamber according to claim 1 or 2,
**characterised in that**
the open end (52) of the cannula (11) for pushing out the glass fibre (09) terminates flush with the inner contour (31) of the sealing ring (22) and the glass fibre (09) pushed out of the cannula (11) is immersed with its measuring tip (28) in an edge region (29) of the substance sample (05) to be investigated.

4. The diffusion chamber according to one or more of the preceding claims,
**characterised in that**
the optical microsensor (07) is fixed in a straight groove (36) in the sample slide (20) by means of an oblique screw connection (38).

5. The diffusion chamber according to one or more of the preceding claims,
**characterised in that**
the measuring tip (28) has a diameter of 150 µm or less.

6. The diffusion chamber according to claim 5,
**characterised in that**
a measuring volume of 20 µl or less for the substance sample (05) to be investigated is used in the sample chamber (04).

7. The diffusion chamber according to one or more of the preceding claims,
**characterised in that**
the optical microsensor (07) comprises a measurement control for ascertaining at least one measurement value per second.

8. The diffusion chamber according to one or more of the preceding claims,
**characterised in that**
the spectrophotometer (13) comprises a measurement control for ascertaining at least 1000 spectra per second in a continuous wavelength range between 200 nm and 1100 nm.

9. The diffusion chamber according to one or more of the preceding claims,
**characterised in that**
the spectrophotometer (13) comprises a halogen- or luminescent-light source (15) for irradiating the substance sample (05) to be investigated.

10. The diffusion chamber according to one or more of the preceding claims,
**characterised in that**
the sample chamber (04) is temperature-regulated with distilled water or water enriched with glycol.

11. The diffusion chamber according to one or more of the preceding claims,
**characterised in that**
an adjustable gas mixture, for example of nitrogen, oxygen and carbon dioxide, is fed via the gas supply (17) to the sample chamber (04).

12. The diffusion chamber according to one or more of the preceding claims,
**characterised in that**
further measuring tips of further optical microsensors, for example for measuring the O₂, CO₂ or NO content, or of chemical microsensors, for example for detecting colour-sensitive compounds, of the substance sample (05) to be investigated are introduced into the sample chamber (04).

13. The diffusion chamber according to one or more of the preceding claims,
**characterised in that**
a substance of biological origin, for example blood or a cell suspension, is selected for the substance sample (05).

## Revendications

1. Chambre de diffusion (01) destinée à déterminer différents paramètres d'une substance aqueuse avec un cylindre (03) central vertical, avec au moins une chambre d'échantillon (04) destinée à recevoir un échantillon de substance (05), avec un capteur de mesure de pH (07) en forme d'aiguille, avec une pointe de mesure (28) contractant l'échantillon de substance, un spectrophotomètre (13) avec une optique en fibre de verre (12, 14) radiographiant l'échantillon de substance et une alimentation de gaz (17) vers la chambre d'échantillon pour la gazéification variable de l'échantillon de substance pendant la détermination des paramètres,
**caractérisée en ce que**
• radialement dans le cylindre central (03), de manière étanche au gaz est inséré un chariot d'échantillons (20) qui comporte une ouverture (21) cylindrique, au-dessus de laquelle une bague d'étanchéité (22) cylindrique, entourant la chambre d'échantillons (04) est placée sur le chariot d'échantillons (20) et comporte une rainure (32) s'étendant en diagonale, dans laquelle est insérée une canule (11) du capteur de mesure de pH conçu en tant que micro-capteur (07) optique en forme de seringue, une fibre optique (09) portant la pointe de mesure (28) étant logée de manière déplaçable dans la canule (11) et la canule (11) étant enfilée de manière étanche au gaz à travers la bague d'étanchéité (22) et **en ce**
• **qu'**axialement dans le cylindre central (03), au-dessus de la chambre d'échantillons (04), un porte-optique (23) cylindrique supérieur, avec une fibre optique (14) amenant la lumière et une lentille de collimateur (24) supérieure et en-dessous de la chambre d'échantillons (04), un porte-optique (25) cylindrique inférieur, avec une fibre optique (12) détournant la lumière, une lentille de collimateur (26) inférieure et une bague d'écartement (27) sont insérés de manière étanche au gaz, par la bague d'écartement (27) insérée, la bague d'étanchéité (22) étant pressée sur le chariot d'échantillons (20) contre le cylindre central (03) et la chambre d'échantillons (04) étant fermée de manière étanche aux gaz provenant de l'environnement de la chambre de diffusion (01) et un écart minimal entre la lentille de collimateur (26) inférieure et l'échantillon de substance (05) étant respecté.

2. Chambre de diffusion selon la revendication 1,
**caractérisée en ce que**
la canule (11) est recourbée de manière doublement angulaire (33, 34), de sorte que l'extrémité (52) ouverte de la canule (11) destinée à pousser vers l'extérieur la fibre de verre (09) soit déplacée en parallèle.

3. Chambre de diffusion selon la revendication 1 ou 2,
**caractérisée en ce que**
l'extrémité (52) ouverte de la canule (11) destinée à pousser vers l'extérieur la fibre de verre (09) se termine à fleur du contour antérieur (31) de la bague d'étanchéité (22) et par sa pointe de mesure (28), la fibre de verre (09) poussée hors la canule (11) est plongée dans une zone marginale (29) de l'échantillon de substance (05) à analyser.

4. Chambre de diffusion selon l'une quelconque ou plusieurs des revendications précédentes,
**caractérisée en ce que**
le micro-capteur (07) optique est fixé par un vissage (38) diagonal dans une rainure (36) dans le chariot d'échantillons (20).

5. Chambre de diffusion selon l'une ou plusieurs des revendications précédentes,
**caractérisée en ce que**
la pointe de mesure (28) présente un diamètre de 150 µm ou inférieur.

6. Chambre de diffusion selon la revendication 5,
**caractérisée en ce que**
dans la chambre d'échantillons (04) est inséré un volume de mesure de 20 µl ou inférieur pour l'échantillon de substance (05) à analyser.

7. Chambre de diffusion selon l'une quelconque ou plusieurs des revendications précédentes,
**caractérisée en ce que**
le micro-capteur (07) optique comporte une commande de mesure destinée à déterminer au moins une valeur de mesure par seconde.

8. Chambre de diffusion selon l'une quelconque ou plusieurs des revendications précédentes,
**caractérisée en ce que**
le spectrophotomètre (13) comporte une commande de mesure destinée à déterminer au moins 1000 spectres par seconde dans une plage continue de longueurs d'ondes comprise entre 200 nm et 1100 nm.

9. Chambre de diffusion selon l'une quelconque ou plusieurs des revendications précédentes,
**caractérisée en ce que**
le spectrophotomètre (13) comporte une source lumineuse (15) halogène ou luminescente destinée à irradier l'échantillon de substance (05) à analyser.

10. Chambre de diffusion selon l'une quelconque ou plusieurs des revendications précédentes,
**caractérisée en ce que**
la chambre d'échantillons (04) est tempérée avec de l'eau distillée ou enrichie de glycol.

11. Chambre de diffusion selon l'une quelconque ou plusieurs des revendications précédentes,
**caractérisée en ce que**
par l'intermédiaire de l'alimentation de gaz (17), un mélange gazeux réglable, composé par exemple d'azote, d'oxygène et de dioxyde de carbone est alimenté vers la chambre d'échantillons (04).

12. Chambre de diffusion selon l'une quelconque ou plusieurs des revendications précédentes,
**caractérisée en ce que**
dans la chambre d'échantillons (04) sont introduites des pointes de mesure supplémentaires de micro-capteurs optiques supplémentaires, par exemple pour la mesure de la teneur en O₂, en CO₂ ou en NO ou de micro-capteurs chimiques supplémentaires, par exemple pour la détection de composés sensibles à la couleur de l'échantillon de substances (05) à analyser.

13. Chambre de diffusion selon l'une quelconque ou plusieurs des revendications précédentes,
**caractérisée en ce que**
pour l'échantillon de substance (05), il est sélectionné une substance d'origine biologique, par exemple du sang ou une suspension cellulaire.
